(19)

Europäisches
Patentamt
European
Patent Office
Office européen
des brevets

(11)　**EP 3 427 641 A1**

(12)　**EUROPEAN PATENT APPLICATION**

(43) Date of publication:
**16.01.2019　Bulletin 2019/03**

(51) Int Cl.:
***A61B 5/00*** *(2006.01)*

(21) Application number: **18162336.4**

(22) Date of filing: **16.03.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:　**11.07.2017　JP 2017135616**

(71) Applicant: **Hitachi, Ltd.**
**Chiyoda-ku**
**Tokyo 100-8280 (JP)**

(72) Inventors:
 • **TANAKA, Tomohiko**
  **Tokyo, 100-8280 (JP)**
 • **TAKEZAKI, Taiichi**
  **Tokyo, 100-8280 (JP)**
 • **IMAI, Ryo**
  **Tokyo, 100-8280 (JP)**

 • **SEO, Yoshiho**
  **Tokyo, 100-8280 (JP)**
 • **MATSUDA, Takahiro**
  **Tokyo, 100-8280 (JP)**
 • **ONOE, Shinsuke**
  **Tokyo, 100-8280 (JP)**
 • **SAKAMOTO, Takeshi**
  **Tokyo, 100-8280 (JP)**
 • **TANAKA, Kimio**
  **Tokyo, 100-8280 (JP)**
 • **KAWAMURA, Ryo**
  **Tokyo, 100-8280 (JP)**
 • **SEYA, Yoshiyuki**
  **Tokyo, 100-8280 (JP)**
 • **WATANABE, Yutaka**
  **Tokyo, 100-8280 (JP)**

(74) Representative: **Strehl Schübel-Hopf & Partner**
**Maximilianstrasse 54**
**80538 München (DE)**

(54)　**PHOTOACOUSTIC CATHETER SYSTEM AND CONTROL METHOD OF PHOTOACOUSTIC CATHETER SYSTEM**

(57)　Treatment with an acoustic catheter is facilitated. A photoacoustic catheter system (C, C1) includes: an imaging laser beam generator (2) that generates an imaging laser beam (R1) used for imaging; a treatment laser beam generator (3) that generates a treatment laser beam (R2) used for treatment; a driver (19) that provides driving to cause the imaging laser beam (R1) and the treatment laser beam (R2) to be emitted in a predetermined direction with respect to an advancing direction of a catheter; an acoustic element (18) that receives an acoustic wave generated due to irradiation of the imaging laser beam (R1); and a treatment laser beam controller (7), wherein the treatment laser beam controller (7) synchronously emits the imaging laser beam (R1) and the treatment laser beam (R2) so as to be directionally aligned with each other.

FIG. 3

EP 3 427 641 A1

## Description

### TECHNICAL FIELD

The present invention relates to a technique of a photoacoustic catheter system using a photoacoustic catheter, and a control method of the same.

### BACKGROUND ART

Imaging a blood vessel broadly by X-ray fluoroscopy is provided in order to identify a lesion for treating vascular stenosis or the like. However, there is a problem that such imaging undesirably causes radiation exposure. Intravascular catheter imaging with light or ultrasound is utilized as a method to solve this problem. Intravascular catheter imaging allows for reducing burden on patients and taking local images. In addition, catheter treatment for vascular stenosis gives fewer burden on patients than open chest surgery, and therefore tends to be increasingly utilized.

As a technique of such intravascular catheter imaging, Patent Document 1 discloses an optical imaging prove that "includes: an optical fiber that transmits light between a front end and a rear end of the probe, and has a condenser lens on the leading end side thereof; a piezoelectric element or an electrostrictive element that causes the optical fiber near the condenser lens to make an angle with respect to its axis line; and an optical path changer that is collinearly arranged in front of the condenser lens, wherein the optical path changer changes an radiation angle of a light beam radiated through the condenser lens to cause the light beam to be radiated stereoscopically, achieving three-dimensional scanning."

Surgery in an extremely small area such as catheter treatment requires both (1) visibility and (2) operability. Particularly, in cases such as chronic total occlusion (CTO), it is required to capture an image of the lesion in real time and then to accurately provide device treatment to the area specified by the operator (physician).

Here, a description will be given of visibility and operability required for catheter treatment.

(1) Visibility: For vascular stenosis such as CTO, visual recognition in front of the catheter is important.
(2) Operability: CTO treatment using a catheter usually involves piercing a guide wire into the lesion under X-ray fluoroscopy. However, under X-ray fluoroscopy, it is difficult to determine the actual lesion. Alternatively, there is treatment using a laser, but laser beam treatment needs to have the lesion identified. In addition, even when the lesion is identified, extremely precise positional accuracy is required to irradiate the lesion with laser beams.

### PRIOR ART DOCUMENT

**Patent Document**

Patent Document 1: International Patent Application Publication No. 2016/063406

### SUMMARY OF THE INVENTION

**Problems to be solved**

As described above under (1) visibility, for a CTO case, a field of view in front of the catheter needs to be made visible to identify the occlusive lesion for treating the identified lesion. However, no catheter has been put into practical use that makes a field of view in front thereof visible. Particularly, no catheter has been put into practical use that makes a field of view in front thereof visible and is used for laser beam treatment. Further, with regard to (2) operability, no technique of laser beam treatment using a catheter has been disclosed so far.

The present invention has been made in view of such a background, and the present invention is intended to facilitate treatment with an acoustic catheter.

**Solution to Problems**

In order to solve the above-mentioned problems, the present invention provides a photoacoustic catheter system including: an imaging laser beam generator that generates an imaging laser beam used for imaging; a treatment laser beam generator that generates a treatment laser beam used for treatment; an emitter that emits the imaging laser beam and the treatment laser beam so as to be directionally aligned with each other; a driver that drives the emitter so as to emit the imaging laser beam and the treatment laser beam toward a predetermined direction with respect to an advancing direction of a catheter; an acoustic detector that receives an acoustic wave generated due to irradiation of the imaging laser beam; and a controller that causes the emitter to synchronously emit the imaging laser beam and the treatment laser beam. Other solutions will be described as appropriate in respective embodiments.

**Advantageous Effects of the Invention**

The present invention facilitates treatment with an acoustic catheter.

**BRIEF DESCRIPTION OF DRAWINGS**

FIG. 1 is a diagram showing a front end of a catheter 1 used in a first embodiment;
FIG. 2 is a schematic diagram showing a laser-beam emission mechanism at the front end of the catheter 1;
FIG. 3 is a functional block diagram of a photoacoustic catheter system C according to the first embodiment;
FIG. 4 is a flowchart of a procedure by the photoacoustic catheter system C executed in the first embodiment;
FIG. 5 is a flowchart of a detailed procedure of emitting an imaging laser beam (step S2 in FIG. 4) executed in the first embodiment;
FIG. 6 is a diagram showing an example of a waveform of a driving voltage applied to a driver 19;
FIG. 7 is a front view (in the axial direction of the catheter 1) of the driver 19 (driving device 14);
FIG. 8 is a diagram showing a waveform of a voltage applied to the driver 19 and emission timings of the imaging laser beam;
FIG. 9 is a diagram illustrating an emission trail of an imaging laser beam R1 caused by applying the voltage shown in FIG. 8;
FIG. 10 is an enlarged view of the vicinity of the origin in FIG. 9;
FIG. 11 is a flowchart of a detailed procedure of address correction processing (step S5 in FIG. 4) executed in the first embodiment;
FIG. 12 is a schematic diagram showing an emission position in a normal state (state without distortion);
FIG. 13 is a schematic diagram showing a distorted emission position;
FIG. 14 is a flowchart of a detailed procedure of image processing (step S6 in FIG. 4) executed in the first embodiment;
FIG. 15 is a diagram showing a distance between an acoustic element 11 and an object irradiated by the imaging laser beam R1;
FIG. 16 is a diagram showing a temporal change in signal intensity detected by the acoustic element 18 in FIG. 15;
FIG. 17 is a flowchart of a detailed procedure of lesion identification processing (step S13 in FIG. 4) to be executed in the first embodiment;
FIG. 18 is a flowchart of a detailed procedure of treatment laser beam emission processing (step S14 in FIG. 4) executed in the first embodiment;
FIG. 19 is a diagram showing emission timings of the treatment laser beam R2;
FIG. 20 is a diagram showing an imaged region;
FIG. 21 is a view showing an example of a catheter-captured image by the photoacoustic catheter system C according to the first embodiment;
FIG. 22 is a functional block diagram of a photoacoustic catheter system C1 according to a second embodiment;
FIG. 23 is a diagram of an imaging laser beam generator 2a used in a third embodiment; and
FIG. 24 is a diagram illustrating an example of an interface device 6 used in a fourth embodiment.

**DETAILED DESCRIPTION**

Next, a description will be given in detail of embodiments of the present invention, with reference to the drawings as appropriate.

**First Embodiment**

<Structure of Catheter 1 >

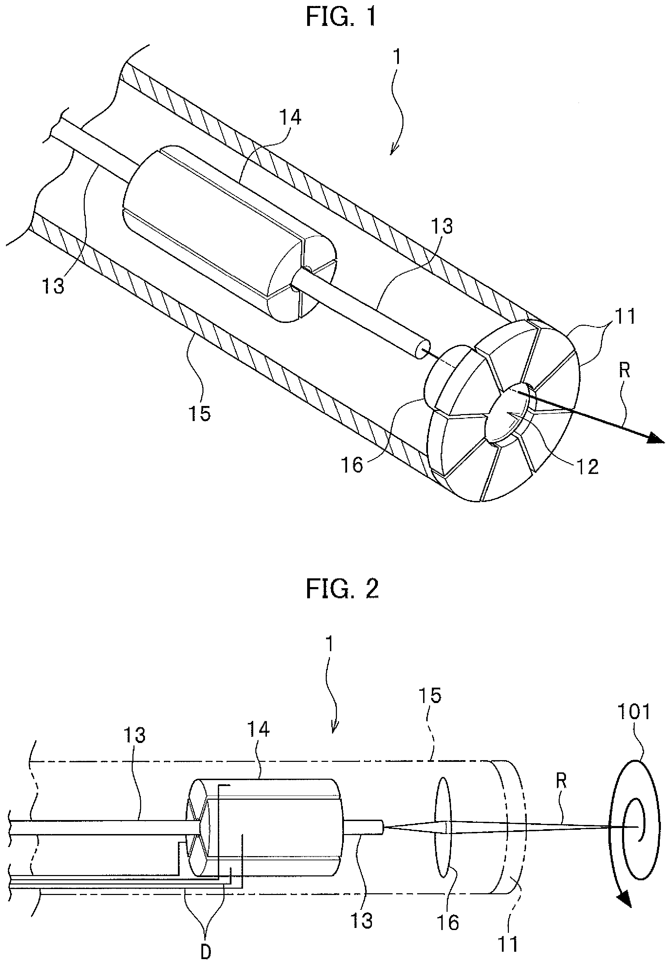

FIG. 1 is a diagram showing a front end of a catheter 1 used in a first embodiment. FIG. 2 is a schematic diagram showing a laser-beam emission mechanism at the front end of the catheter 1. Note that in the present application, having a laser beam R from an optical fiber 13 is referred to as "emitting," and having the emitted laser beam R on an observation object is referred to as "irradiating." As shown in FIG. 1, a photoacoustic catheter 1 (hereinafter simply referred to as a catheter 1) has a plurality of acoustic elements (acoustic detectors) 11 arranged in a ring shape at the front end of the catheter 1 itself. Here, the acoustic element 11 is composed of a piezoelectric element or the like manufactured in MEMS (Micro Electro Mechanical Systems) technology. The acoustic element 11 may be a single element or may be of an array type having a plurality of elements mounted as shown in FIG. 1. Making the acoustic element 11 be of an array type having the plurality of elements mounted allows for utilizing a Delay-and-Sum technique. Therefore, a detected signal can be enhanced by the Delay-and-Sum technique to sharpen an image captured by the catheter (hereinafter, referred to as a catheter-captured image).

A hollow 12 is formed inside the acoustic element 11 arranged in a ring shape, and the laser beam R emitted from the optical fiber 13 passes through the hollow 12. Once the laser beam R emitted from the optical fiber 13 in the catheter 1 passes through the hollow 12 to cause an observation object of a living body to be irradiated with the laser beam R, the observation object produces heat to expand itself in volume. This volume expansion causes an acoustic wave to be generated, and this acoustic wave is detected by the acoustic element 11. That is, the acoustic element 11 receives the acoustic wave generated by the irradiated laser beam R.

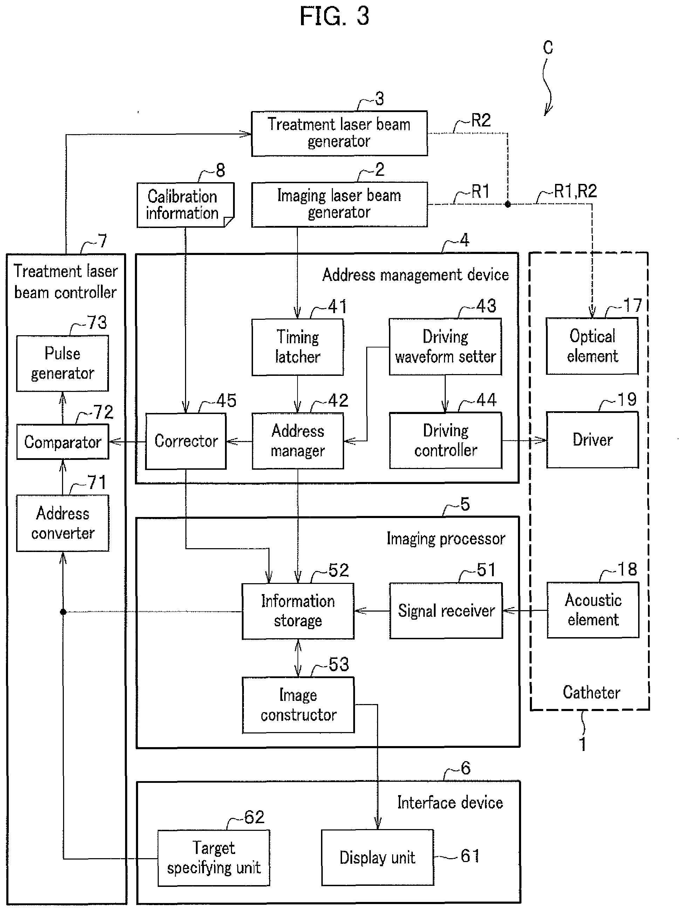

Next, a description will be given of the laser-beam emission mechanism in the catheter 1 used in the first embodiment. In the catheter 1, the laser beam R is transmitted from an imaging laser beam generator 2 or a treatment laser beam generator 3 (see FIG. 3) through the optical fiber 13. A treatment laser beam R2 (see FIG. 3) may be the same laser beam R as an imaging laser beam R1 (see FIG. 3). In this case, power of the laser beam R is changed to switch the imaging laser beam R1 and the treatment laser beam R2. This requires only one laser beam generator, allowing for reducing mafufacturing costs. As shown in FIGS. 1 and 2, a driver 14 preferably uses a piezoelectric element such as a four-pole PZT element (hereinafter simply referred to as PZT element) in a cylindrical shape. As shown in FIG. 2, a potential difference is applied, via electric leads D, between the opposing electrodes in the PZT element to bend the PZT element toward a direction following the potential diffference. The voltage applied to two pairs of opposing electrodes is made to have a sinusoidal wave, and its phase is shifted by $\pi/2$ to swing a front end of the optical fiber 13 passing through the PZT element as shown in FIG. 2. Note that the radius of swing of the optical fiber 13 (the radius of swing shown in FIG. 2) is controlled by an amplitude of the sine wave voltage applied to the PZT element. The amplitude of the voltage applied to the PZT element is changed to cause the laser beam R emitted from the catheter 1 to draw a trail 101 in a spiral shape. At this time, a frequency of swinging the laser beam R is, for example, 8 kHz. In this way, the driver 14 drives the PZT emlemet to emit the laser beam R toward a predetermined direction with respect to the advancing direction of the catheter 1.

The laser beam R emitted from the front end of the optical fiber 13 diverges at an angle specific to the optical fiber 13. Therefore, as shown in FIGS. 1 and 2, a lens 16 is provided for converging a laser beam to the observation object.

In addition, as shown in FIGS. 1 and 2, these elements are covered by a cover 15 to assume a mechanical property of the catheter 1.

Note that although not shown in FIG. 1, the catheter 1 may include a guide wire and/or a flushing mechanism. Flushing is to wash blood or the like with water. Including the guide wire allows for providing a treatment using not only the laser beam R but also the guide wire, depending on the situation. Additionally, the catheter 1 may include a tubular structure (liquid injector) (not shown) to inject a transparent liquid for provisionally removing blood into the catheter 1. This allows blood in the blood vessel to be provisionally removed to obtain a favorable catheter-captured image.

<System block diagram>

FIG. 3 is a functional block diagram of a photoacoustic catheter system C according to the first embodiment. The photoacoustic catheter system C includes the catheter 1, the imaging laser beam generator 2, and the treatment laser beam generator 3. In addition, the photoacoustic catheter system C includes an address management device (controller) 4, an imaging processor 5, an interface device (input/output device) 6, and a treatment laser beam controller 7.

The imaging laser beam generator 2 generates the imaging laser beam R1 which is a low power pulse laser beam for imaging. The treatment laser beam generator 3 generates the treatment laser beam R2 which is a high power pulse laser beam for treatment. Both the imaging laser beam R1 and the treatment laser beam R2 travel inside the optical fiber 13. Note that in FIG. 3, a broken line arrow indicates the laser beam R (see FIGS. 1 and 2).

The catheter 1 has an optical element 17, a driver 19, and an acoustic element (acoustic detector) 18. The optical element 17 includes the front end of the optical fiber 13 and the lens 16, and emits the imaging laser beam R1 and the treatment laser beam R2. The driver 19 is the driver 14 in FIGS. 1 and 2, which has already been described with reference to FIGS. 1 and 2, so that a description thereof will be omitted here. Also, the acoustic element 18 is the acoustic element 11 in FIGS. 1 and 2, and therefore a description thereof will be omitted here.

The address management device 4 manages a timing, at which the imaging laser beam R1 has been emitted, as an address. The address indicates a position (emission position) at which the imaging laser beam beam R1 has been emitted, and is represented such as by coordinates. The address management device 4 includes a timing latcher 41, an address manager 42, a driving waveform setter 43, a driving controller 44, and a corrector 45. The timing latcher 41 records an emission timing of the imaging laser beam R1 based on information from the imaging laser beam generator 2.

The address manager 42 calculates the address at which the imaging laser beam R1 has been emitted, based on the emission timing recorded by the timing latcher 41 and the driving voltage waveform set by the driving waveform setter 43. The driving waveform setter 43 sets the driving voltage waveform. The driving controller 44 applies driving voltage to the driver 19 according to the driving voltage waveform set by the driving waveform setter 43. The corrector 45 corrects the address calculated by the address manager 42, based on calibration information (distortion information) 8 inputted in advance such as by manual input, and the like. As a result, the corrector 45 generates a corrected address. Processing by the corrector 45 will be described later.

The imaging processor 5 reconstructs the catheter-captured image, based on a signal transmitted from the acoustic element 18, the corrected address, and the like. The imaging processor 5 includes a signal receiver 51, an information storage 52, and an image constructor 53. The signal receiver 51 receives the signal transmitted from the acoustic element 18. The information storage 52 stores the signal received by the signal receiver 51, the address of the emission timing of the imaging laser beam sent from the address manager 42, the corrected address calculated by the corrector 45, and the like. The image constructor 53 reconstructs the catheter-captured image, based on the information stored in the information storage 52, and the like. Note that in the present embodiment, the signal received by the signal receiver 51, the address of the emission timing of the imaging laser beam sent from the address manager 42, the corrected address, and the like are once stored in the information storage 52, and then retrieved by the image constructor 53. However, the signal received by the signal receiver 51, the address of the emission timing of the imaging laser beam sent from the address manager 42, the corrected address, and the like may directly be inputted to the image constructor 53 without being stored in the information storage 52. Additionally, the image constructor 53 stores the reconstructed catheter-captured image in the information storage 52.

The interface device 6 provides input and output. The interface device 6 has a display unit 61 and a target specifying unit 62. The display unit 61 displays the catheter-captured image reconstructed by the image constructor 53. The target specifying unit 62 is composed of a pointing device or the like. The operator (such as a nurse) specifies a lesion to be treated in the catheter-captured image, which is displayed on the display unit 61, with the target specifying unit 62.

The treatment laser beam controller 7 controls emitting the treatment laser beam R2. The treatment laser beam controller 7 has an address converter 71, a comparator 72, and a pulse generator 73. The address converter 71 converts a target on the catheter-captured image specified by the target specifying unit 62 into an address (target address), based on the catheter-captured image, the address, and the like which are stored in the information storage 52. The comparator 72 compares the address (current address) at which imaging is currently being made (the imaging laser beam R1 is being emitted) with the target address sent from the address converter 71. A corrected address may be used as the current address. Note that imaging continues even during treatment. When the comparator 72 determines that the current address matches the target address, the pulse generator 73 sends a pulse for emitting the treatment laser beam R2 to the treatment laser beam generator 3. This causes the treatment laser beam generator 3 to emit the treatment laser beam R2 at the timing when the pulse generator 73 has generated a pulse.

<Flowcharts>

<<Overall Processing>>

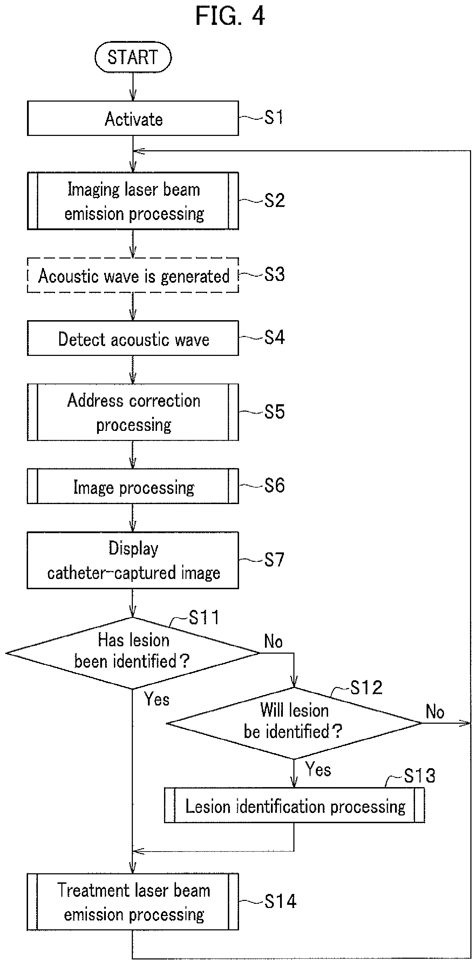

FIG. 4 is a flowchart showing a procedure by the photoacoustic catheter system C execued in the first embodiment. Note that processing indicated by a broken line in FIG. 4 is provided other than in the photoacoustic catheter system C. In the drawings to be referred to hereinbelow, FIG. 3 is referred to as appropriate. First, the operator activates each part of the photoacoustic catheter system C via the interface device 6 (S1). Next, imaging laser beam emission processing is executed to emit the imaging laser beam R1 (S2). Details of the imaging laser beam emission processing will be described later. The emitted imaging laser beam R1 is irradiated on the object. The object absorbs the imaging laser beam R1 to thermally expands instantaneously, generating an acoustic wave (S3).

Then, the acoustic element 18 detects the acoustic wave from the object (S4). Upon receiving the acoustic wave, the acoustic element 18 generates a voltage having a magnitude depending on the detected acoustic wave. The generated voltage is converted into a digital signal having a predetermined magnitude by the signal receiver 51 equipped with an amplifier and an ADC (Analogue-Digital Converter), which are not shown. The converted digital signal is stored in the information storage 52. Note that the acoustic element 18 may be arrayed using a plurality of channels and data may be stored for each channel.

Upon receiving the acoustic wave, the acoustic element 18 transmits an electric signal with a voltage depending on the magnitude of the acoustic wave. The transmitted electric signal is received by the imaging processor 5. Next, the address management device 4 executes address correction processing (S5). Details of the address correction processing will be described later. Then, the imaging processor 5 uses the result of the address correction processing (corrected address) to execute image processing (S6). Details of the image processing will be described later. Then, the catheter-captured image, which is outputted as a result of the image processing, is displayed on the display unit 61 (S7).

Next, the user (physician or the like) determines whether or not the lesion has been identified (S11). If the lesion has not been identified as a result of step S11 (No in S11), the user (physician or the like) determines whether or not a lesion will be identified (S12). If a lesion will be identified, the user (nurse or the like), for example, selectively inputs an "identify lesion" button displayed on the display unit 61.

If a lesion will not be identified as a result of step S12 (No in S12), the photoacoustic catheter system C returns processing to step S2. If a lesion will be identified as a result of step S12 (Yes in S12), lesion identification processing is executed via the interface device 6 (S13). Details of the lesion identification processing will be described later. Next, the photoacoustic catheter system C advances processing to step S14.

Alternatively, if the lesion has already been identified as a result of step S11 (Yes in S11), the treatment laser beam controller 7 and the treatment laser beam generator 3 execute a treatment laser beam emission processing (S14). Details of the treatment laser beam emission processing will be described later. Next, the photoacoustic catheter system C returns processing to step S2. That is, the photoacoustic catheter system C executes a treatment while capturing images. In other words, the imaging laser beam R1 and the treatment laser beam R2 are emitted from the same optical fiber 13 (coaxially, that is, directionally aligned, or toward the same direction).

Note that being coaxial is preferable for positional accuracy, but cores (not shown) for introducing the treatment laser beam R2 and the imaging laser beam R1 into the optical fiber 13 can separately be arranged from each other. In other words, a core dedicated to the treatment laser beam R2 and a core dedicated to the imaging laser beam R1 can be arranged inside the optical fiber 13. Note that when the cores propagating the treatment laser beam R2 and the imaging laser beam R1 are separately arranged in the optical fiber 13, the imaging laser beam R1 and the treatment laser beam R2 will irradiate different areas. However, the difference is very small and therefore the treatment laser beam R2 is simply required to irradiate a target area (lesion), as will be described later.

The resolution of the catheter-captured image depends on "(the optical magnification of the lens 16) x (the core diameter for the imaging laser beam R1)" and therefore the core of the optical fiber 13 used for the imaging laser beam R1 is preferably thin. On another front, the treatment laser beam R2 generally has higher power than the imaging laser beam R1. This may cause the core (optical fiber 13) to be damaged, if the core diameter of the optical fiber 13 is fixed to fit for the imaging laser beam R1 when the imaging laser beam R1 and the treatment laser beam R2 are emitted coaxially (from the common core). Separately arranging the cores for the imaging laser beam R1 and the treatment laser beam R2 can avoid the risk of the core (optical fiber 13) being damaged, without lowering the resolution.

This allows the operator (physician) to provide treatment while viewing the image in real time. For example, the operator (physician) can proceed with the treatment while confirming whether the coagulated blood or the like is suitably removed by the treatment laser beam R2.

<<Imaging Laser beam Emission Processing>>

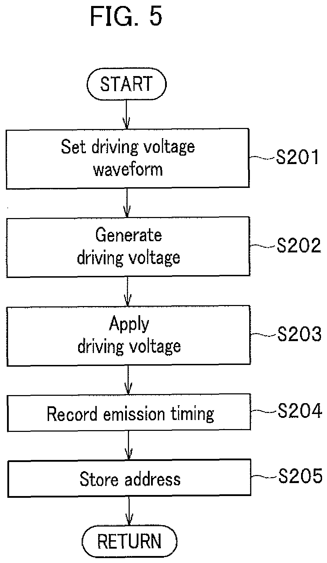

FIG. 5 is a flowchart of the detailed procedure of the imaging laser beam emission processing (step S2 in FIG. 4) executed in the first embodiment. The driving waveform setter 43 sets a driving voltage waveform according to the angular velocity of swinging the optical fiber 13 (S201). Then, the driving controller 44 generates the driving voltage set in step S201 (S202). Subsequently, the driving controller 44 applies the generated driving voltage to the driver 19 (S203). As a result, swinging the optical fiber 13 is started by the driver 19.

Next, the timing latcher 41 records the emission timing of the imaging laser beam R1 (S204). The emission timing is specifically the emission time of the imaging laser beam R1, or the like. A photodetector may be used to store the emission time of the imaging laser beam R1 as the emission timing, or the output time of the synchronization signal may be stored as the emission timing where the signal is outputted at the time of outputting the imaging laser beam R1. The address manager 42 calculates information about the emission timing of the imaging laser beam R1 as an address. Then, the address manager 42 stores the information about the emission timing of the imaging laser beam R1, as an address, in the information storage 52 (S205). Note that the address manager 42 calculates an address based on the driving voltage waveform set by the driving waveform setter 43 and the emission timing.

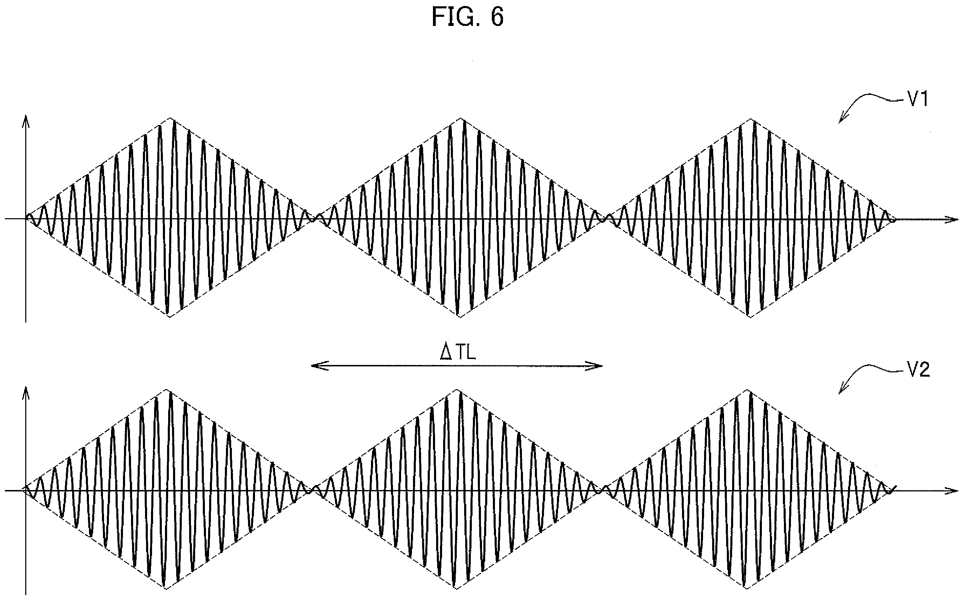

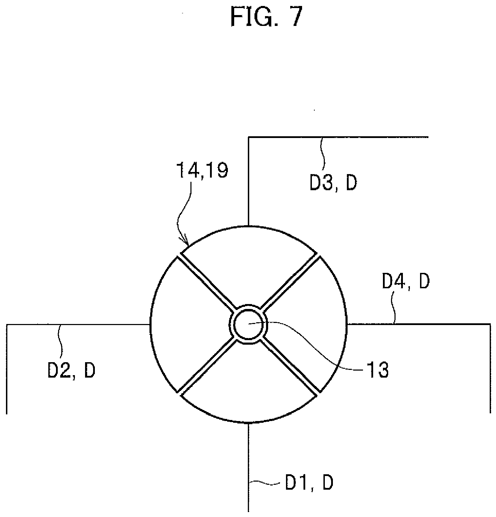

FIG. 6 is a diagram showing an example of the waveform of the driving voltage applied to the driver 19. In addition, FIG. 7 is a front view (in the axial direction of the catheter 1) of the driver 19 (driving device 14). As shown in FIG. 7, the driver 19 is connected with electric leads D1 to D4 (D) circumferentially at an angle of $\pi/2$. Here, a waveform V1 in FIG. 6 is a waveform of a driving voltage applied across the electric leads D1 and D3 in FIG. 7. Additionally, a waveform V2 in FIG. 6 is a waveform of a driving voltage applied across the electric leads D2 and D4 in FIG. 7. Here, the phase of the waveform V1 is shifted by $\pi/2$ from that of the waveform V2. Applying such voltages, having the waveforms V1 and V2, to the driver 19 causes the front end of the optical fiber 13 to draw the trail 101 in a spiral shape, as shown in FIG. 2. Here, $\Delta TL$ in FIG. 6 is a cycle, in the trail 101 in a spiral shape (see FIG. 2), of the front end of the optical fiber 13 expanding the radius of the trail 101 from the center and then returning to the center again.

<<Address Calculation>>

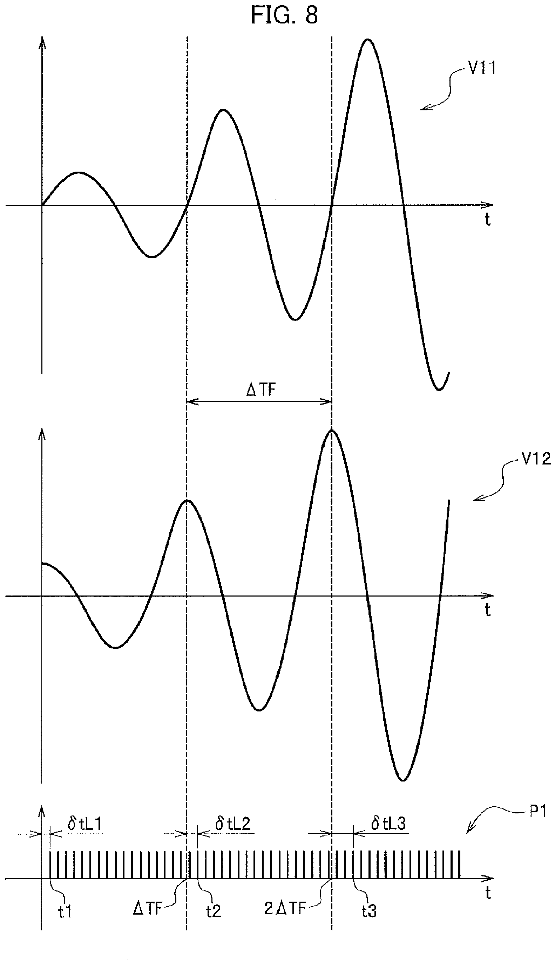

Next, an address calculation will be described with reference to FIGS. 8 to 10. FIG. 8 is a diagram showing a waveform of the voltage applied to the driver 19 and emission timings of the imaging laser beam. Note that a waveform V11 in FIG. 8 is an enlarged view of the waveform V1 in FIG. 6 near time 0. Similarly, a waveform V12 is an enlarged view of the waveform V2 in FIG. 6 near time 0. A timing chart P1 indicates the emission timing of the imaging laser beam R1. Here, the cycle of the voltage waveform is assumed to be $\Delta TF$. A time length from time 0 to an emission timing t1 of the first imaging laser beam is assumed to be $\delta tL1$. In addition, a time length from the time $\Delta TF$ to an emission timing t2 of the second imaging laser beam is assumed to be $\delta tL2$. Further, a time length from the time $2\Delta TF$ to an emission timing t3 of the third imaging laser beam is assumed to be $\delta tL3$.

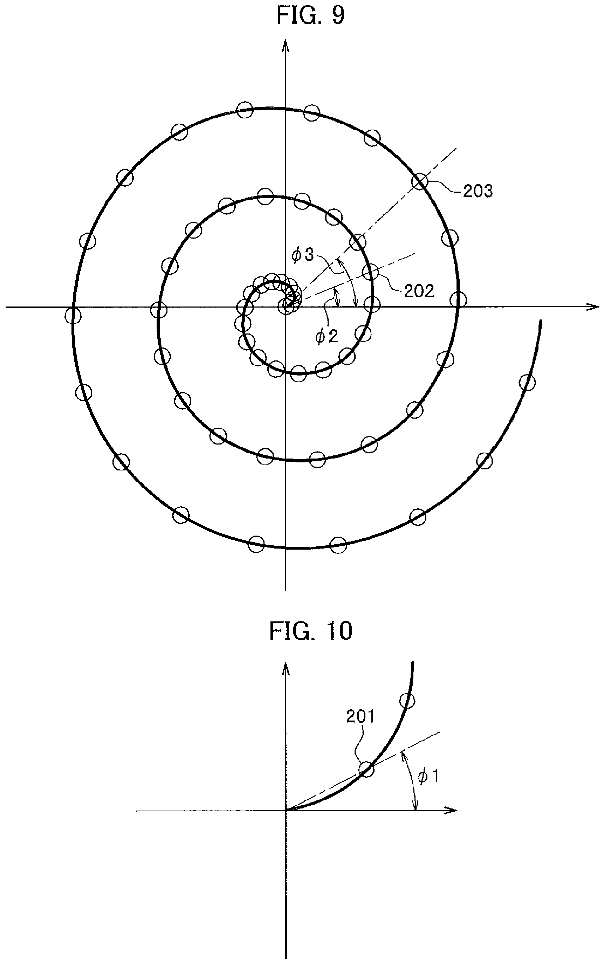

FIG. 9 is a diagram illustrating an emission trail of the imaging laser beam R1 caused by applying the voltage shown in FIG. 8, and FIG. 10 is an enlarged view of the vicinity of the origin in FIG. 9. Here, a reference numeral 201 in FIG. 10 indicates a position of the imaging laser beam R1 being emitted first time since the start of emitting the imaging laser beam R1. That is, it is the emission position (address) of the imaging laser beam R1 emitted at the timing t1 in the timing chart P1 in FIG. 8. As shown in FIGS. 9 and 10, when the X-axis and the Y-axis are set with respect to the emission trail, an angle $\Phi1$ between the reference numeral 201 and the X-axis is expressed by the following equation (1).

$$\Phi1 = 2\pi * \delta tL1 / \Delta TF \qquad ---(1).$$

That is, the image costructor 53 determines that the position captured by the imaging laser beam R1 emitted at the emission timing t1 of the imaging laser beam in FIG. 8 is the position indicated by the reference numeral 201 where a ray extending from the origin crosses the emission trail at the angle $\Phi1$ to the X-axis.

Similarly, rays extending from the origin cross the emission trail at emission positions 202 and 203 of the imaging laser beam R1 emitted at the timings t2 and t3 in FIG. 8, respectively, at angles $\Phi2$ and $\Phi3$ to the X-axis, and the angles $\Phi2$ and $\Phi3$ are represented by the following equations (2) and (3).

$$\Phi2 = 2\pi * \Delta tL2 / \Delta TF \qquad ---(2)$$

$$\Phi3 = 2\pi * \Delta tL3 / \Delta TF \qquad ---(3).$$

In this manner, the image constructer 53 calculates an emission position (address) corresponding to each emission timing of the imaging laser beam shown in the timing chart P1 in FIG. 8, to obtain the address.

<<Address Correction Processing>>

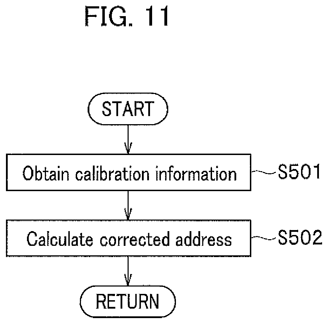

FIG. 11 is a flowchart of a detailed procedure of the address correction processing (step S5 in FIG. 4) executed in the first embodiment. First, the corrector 45 obtains the calibration information 8 (S501). The calibration information 8 is information for calibrating distortion of an image (information about distortion of the emission position of the imaging laser beam R1). The calibration information 8 is information that is inputted in advance via the interface device 6. More specifically, this information is information about deviations caused by swinging of the optical fiber 13, which is obtained during test operation of the photoacoustic catheter system C. The calibration information 8 may be stored in an EEPROM (Electrically Erasable Programmable Read-Only Memory), a server, a USB (Universal Serial Bus) memory or the like. In addition, the following technique is used, for example, to obtain the calibration information 8. First, a calibration kit attached with a calibration point (scale) is mounted on the front end of the catheter 1. Then, a catheter-captured image generated by executing the processing in steps S2 to S4 in FIG. 4 is obtained. The calibration information 8 may be created based on the deviation of the calibration point shown in the obtained catheter-captured image.

Then, the corrector 45 calculates a corrected emission position (corrected address) of the imaging laser beam, based on the calibration information 8 and the address (S502).

<<Calibration Information 8>>

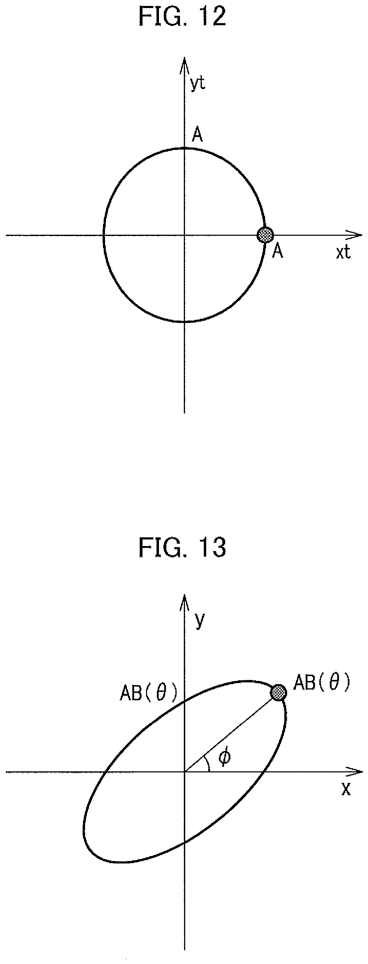

Next, the calibration information 8 will be described with reference to FIGS. 12 and 13. FIG. 12 is a schematic diagram showing an emission position in a normal state (state without distortion). Such emission position of the imaging laser beam R1 is an address. As shown in FIG. 12, it is assumed that an ideal emission position (address) is plotted on a circle having a radius A. Note that the emission position is essentially plotted on a vortex shape (spiral shape), as shown in FIG. 2, but here it is assumed that the shape is circular for the purpose of illustration. An arbitrary emission position (xt, yt) in FIG. 12 is given by following Equation (11). Note that ω is expressed by "ω = dθ/dt."

$$\begin{pmatrix} xt \\ yt \end{pmatrix} = \begin{pmatrix} A\cos(\omega t) \\ A\sin(\omega t) \end{pmatrix} \qquad \text{- - - (11).}$$

FIG. 13 is a schematic diagram showing a distorted emission position. As shown in FIG. 13, it is assumed that the measured emission position is distorted by a phase shift $\Phi$ and by an amplitude distortion B($\theta$), which is angle-dependent, with respect to the emission position in FIG. 12. The phase shift $\Phi$ is derived from a signal delay of the imaging laser beam R1. The amplitude distortion B($\theta$) is derived from a deviation of the waveform of a voltage applied to the driver 19. Note that the phase shift $\Phi$ and the amplitude distortionB($\theta$) are measured in advance as described above. AB ($\theta$) is subject to the constraint that it becomes A when the circumference average is taken (or integrated). At this time, the emission position (address) (xm($\theta$), ym($\theta$)) is expressed by following Equation (12). This address is the address calculated by the address manager 42.

$$AB(\theta) = \begin{pmatrix} xm(\theta) \\ ym(\theta) \end{pmatrix} = \begin{pmatrix} B(\theta)A\cos(\omega t + \phi) \\ B(\theta)A\sin(\omega t + \phi) \end{pmatrix} \qquad \text{- - - (12).}$$

Equation (12) is modified to equations (13) and (14) below.

$$\begin{pmatrix} xm(\theta) \\ ym(\theta) \end{pmatrix} = B(\theta) \begin{pmatrix} \cos\phi & -\sin\phi \\ \sin\phi & \cos\phi \end{pmatrix} \begin{pmatrix} xt \\ yt \end{pmatrix} \qquad \text{- - - (13)}$$

$$\begin{pmatrix} xt \\ yt \end{pmatrix} = \left( B(\theta) \begin{pmatrix} \cos\phi & -\sin\phi \\ \sin\phi & \cos\phi \end{pmatrix} \right)^{-1} \begin{pmatrix} xm(\theta) \\ ym(\theta) \end{pmatrix} \qquad \text{- - - (14).}$$

The inverse matrix in equation (14) is calibration information 8. Note that calculations at a plurality of points are required in order to specify the calibration information 8. In this manner, the signal delay of the imaging laser beam R1 can be corrected. In addition, if a catheter-captured image is displayed on the display unit 61 simply using the emission position (xm (θ), ym (θ)) (that is, the irradiation position) as shown in FIG. 13, the image is displayed in a distorted shape to make it less recognized by the operator. Then, the calibration information 8 in Equation (14) is used to display the image in a suitable shape as a shape to be displayed on the display unit 61. That is, the irradiation position of the imaging laser beam R1 can be calibrated. Note that using the calibration information 8 in Equation (14) causes a deviation between the actually emitted position (that is, the irradiation position) and the position on the catheter-captured image displayed on the display unit 61. However, B ($\theta$) is generally small enough to cause no problem. Note that B ($\theta$) in FIG. 13 has a large value for easy understanding.

<<Image Pprocessing>>

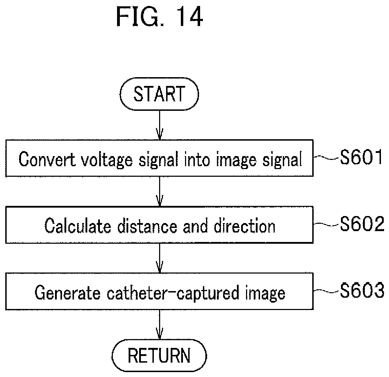

FIG. 14 is a flowchart of a detailed procedure of the image processing (step S6 in FIG. 4) executed in the first embodiment. The image constructor 53 converts the voltage signal stored in the information storage 52 into an image signal (S601). The conversion to the image signal may be any one of Hilbert transformation, Quadrature detection, Back projection and Absolute value computation.

Next, the image constructor 53 calculates the distance and direction from the object to be irradiated with the imaging laser beam R1 to the acoustic element 18 (S602), based on the corrected address and the like calculated by the corrector 45.

Next, the image constructor 53 generates a catheter-captured image (S603), based on the image signal and the corrected emission position (corrected address) of the imaging laser beam R1 stored in the information storage 52. The image may be in 1D (Dimension), 2D, or 3D. The photoacoustic catheter system C generates the catheter-captured image, based on the corrected address, to calibrate the catheter-captured image captured by the imaging laser beam R1, according to the calibration information 8. The image constructor 53 stores the generated catheter-captured image in the information storage 52.

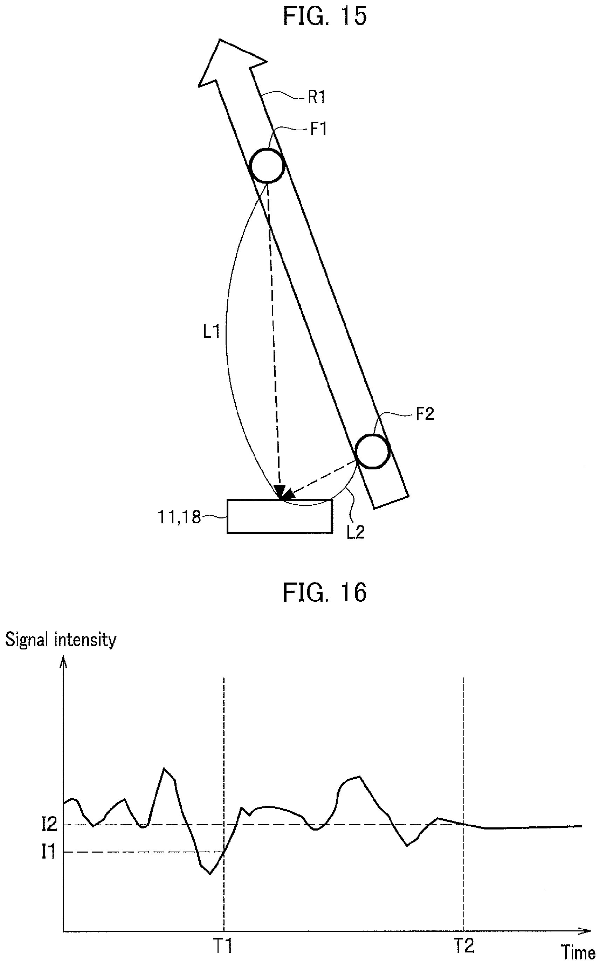

Here, a description will be given of a method of calculating the distance and direction from the object to the acoustic element 18, with reference to FIGS. 15 and 16. It is assumed that the imaging laser beam R1 is emitted toward a direction shown in FIG. 15. It is also assumed that an object F1 and an object F2 exist in the traveling direction of the imaging laser beam R1. The imaging laser beam R1 travels at the light velocity, and therefore the time the imaging laser beam R1 takes since it has been emitted until it reaches the object F1 is equal to that since it has been emitted until it reaches the object F2. In other words, it can be assumed that the time the imaging laser beam R1 takes since it has been emitted until it reaches the object F1 and the time since it has been emitted until it reaches the object F2 are respectively zero. Accordingly, a distance L1 to the object F1 and a distance L2 to the object F2 from the acoustic element 18 (acoustic element 11) can be expressed by the following expressions.

$$L1 = Vs \times T1$$

$$L2 = Vs \times T2.$$

Here, Vs is the speed of sound. T1 is the time since the imaging laser beam beam R1 has been emitted until an acoustic wave is detected by the acoustic element 18. Similarly, T2 is the time since the imaging laser beam R1 has been emitted until an acoustic wave is detected by the acoustic element 18.

FIG. 16 is a diagram showing a temporal change in signal intensity detected by the acoustic element 18 shown in FIG. 15. In FIG. 16, the horizontal axis indicates time and the vertical axis indicates signal intensity (acoustic wave intensity). In addition, time 0 in FIG. 16 is the emisstion time of the imaging laser beam R1. Time T1 and time T2 are the times at which the acoustic waves emitted from the objects F1 and F2 in FIG. 15 respectively reach the acoustic element 18. That is, a signal intensity I1 at time T1 is information about the object F1 (see FIG. 15) located at the distance L1 in FIG. 15. Similarly, a signal intensity I 2 at time T2 is information about the object F2 (see FIG. 15) located at the distance L2 in FIG. 15. Note that directions of the detected objects F1 and F2 with respect to the acoustic element 18 can easily be calculated from the emission direction (that is, the address) of the imaging laser beam R1. In this manner, image data is reconstructed. Note that in the case where a plurality of the acoustic elements 18 are arranged, the image constructor 53 may utilize Delay-and-Sum technique to enhance the detected signal, as described above.

«Lesion Identification Processing»

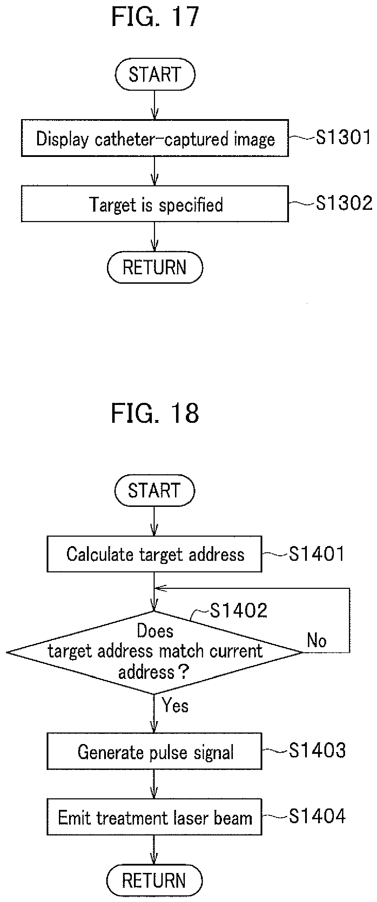

FIG. 17 is a flowchart showing a detailed procedure of the lesion identification processing (step S13 in FIG. 4) executed in the first embodiment. First, a catheter-captured image constructed by the image constructor 53 is displayed on the display unit 61 (S1301). Next, the operator (such as a nurse) specifies a target such as a lesion displayed on the display unit 61 via the target specifying unit 62 (S1302). The target specifying unit 62 has a pointing device or the like. That is, the operator uses the pointing device to specify the target in the image displayed on the display unit 61.

<<Treatment Laser Beam Emission Processing>>

FIG. 18 is a flowchart of a detailed procedure of the treatment laser beam emission processing (step S14 in FIG. 4) executed in the first embodiment. The address converter 71 calculates the emission timing of the imaging laser beam R1 with which the specified target has been captured (target address), based on the catheter-captured image, the address, and the like stored in the information storage 52 (S1401). The comparator 72 determines whether or not the target address calculated in step S1401 matches a current address within a certain margin (S1402). The current address is the address (emission direction, emission position) which the optical fiber 13 currently has. Here, the address (corrected address) corrected by the corrector 45 is used as the current address, but an uncorrected address may be used.

As a result of step S1402, if the target address does not match the current address within a certain margin (No in S1402), the treatment laser beam controller 7 returns processing to step S1402. As a result of step S1402, if the target address matches the current address within a certain margin (Yes in S1402), the pulse generator 73 generates a pulse signal (S1403). Next, the treatment laser beam generator 3 generates the treatment laser beam R2 (high power pulse laser beam) according to the pulse signal (S1404). Note that using a photoacoustic multimode fiber as the optical fiber 13 allows the imaging laser beam R1 and the treatment laser beam R2 to come in, and be emitted from, the single optical fiber 13.

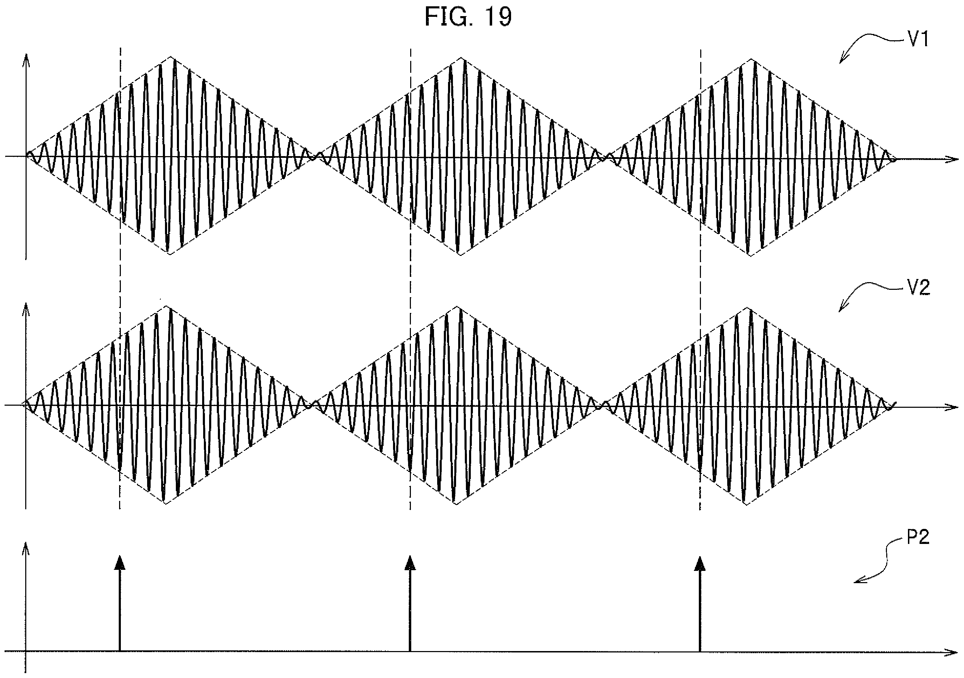

FIG. 19 is a diagram showing emission timings of the treatment laser beam R2. The waveforms V1 and V2 in FIG. 19 are the same as the waveforms V1 and V2 in FIG. 6 to show driving voltage waveforms applied to the driver 19. A reference numeral P2 in FIG. 19 indicates emission timings of the treatment laser beam R2. Note that the horizontal axis indicates time in each chart shown in FIG. 19, and the horizontal axes in respective charts are synchronized. As indicated by the reference numeral P2, a pulse (that is, the treatment laser beam R2) is periodically emitted at a timing when the driving voltage having the waveforms V1 and V2 causes the current address to match the target address.

<Imaged Region>

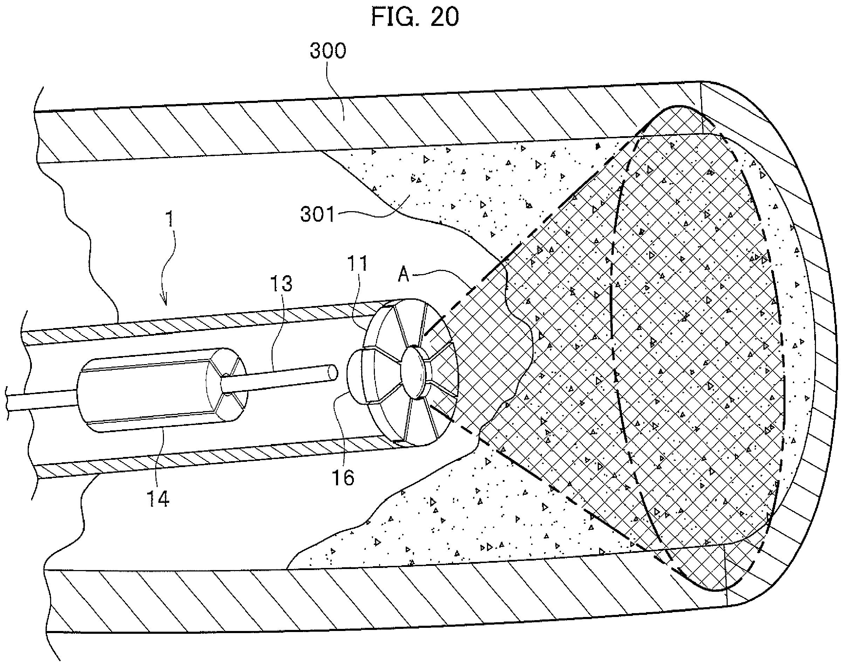

FIG. 20 is a diagram showing an imaged region. As shown in FIG. 20, a region A on a cone indicates an imaged region. A reference numeral 300 denotes a blood vessel developing CTO (chronic total occlusion), and a reference numeral 301 denotes a strictured area of a blood vessel. Note that the blood vessel 300 is shown in cross-section. The structure of the catheter 1 is the same as that in FIG. 1, and then the description thereof will be omitted here.

<Catheter-captured Image>

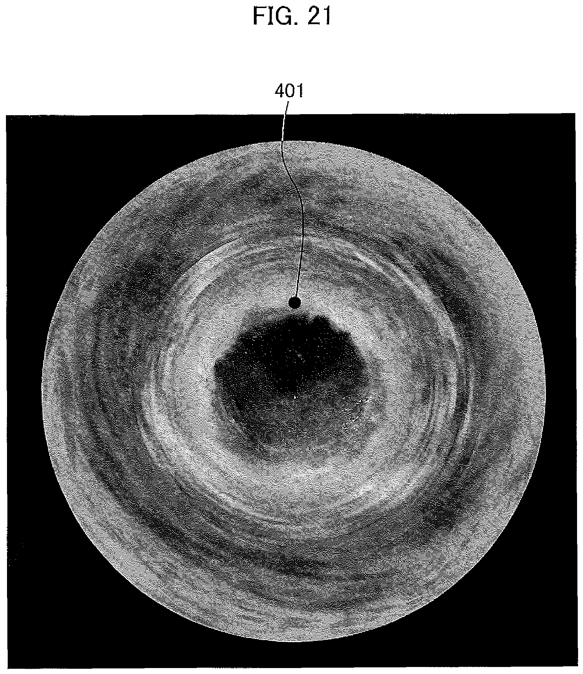

FIG. 21 is a view showing an example of a catheter-captured image by the photoacoustic catheter system C according to the first embodiment. A reference numeral 401 denotes the target specified by the target specifying unit 62 in step S1302 in FIG. 17.
According to the first embodiment, the imaging laser beam R1 and the treatment laser beam R2 are coaxially emitted in synchronization to allow for providing imaging and treatment at the same time. In particular, treatment can be provided while checking is made whether or not a target spot is irradiated with the treatment laser beam R2. That is, the operator (physician) can provide treatment while checking whether or not a desired spot is irradiated with the treatment laser beam R2. In addition, in the first embodiment, the target specifying unit 62 specifies a given area (lesion) on the catheter-captured image, and the treatment laser beam R2 is emitted toward the specified area. In this way, the operator can specify an area to be irradiated with the treatment laser beam R2, while viewing the captured image in real time. When the target is treated, the pulse energy (intensity) of the treatment laser beam R2, the number of times of irradiation, duration of the treatment, and the like are set by the operator on the setting window or the like displayed on the display unit 61, to cause the treatment laser beam controller 7 to implement a laser beam irradiation method desired by the operator. This allows for providing the treatment desired by the operator.
Further, the imaging laser beam R1 is emitted forward of the catheter 1. The front end of the optical fiber 13 draws a voltex (spiral) trail to allow for obtaining an image of an area in the axial direction of the catheter 1 and its surroundings. Furthermore, the photoacoustic catheter system C has the calibration information 8 for calibrating the irradiation position of the imaging laser beam R1. This calibration information 8 is information about distortion of the emission position. Based on this calibration information 8, the address management device 4 calibrates the irradiation position of the imaging laser beam R1. This allows for outputting a catheter-captured image in which distortion of the image due to imperfect swinging of the optical fiber 13, a signal delay of the imaging laser beam R1, and the like have been calibrated.

**Second Embodiment**

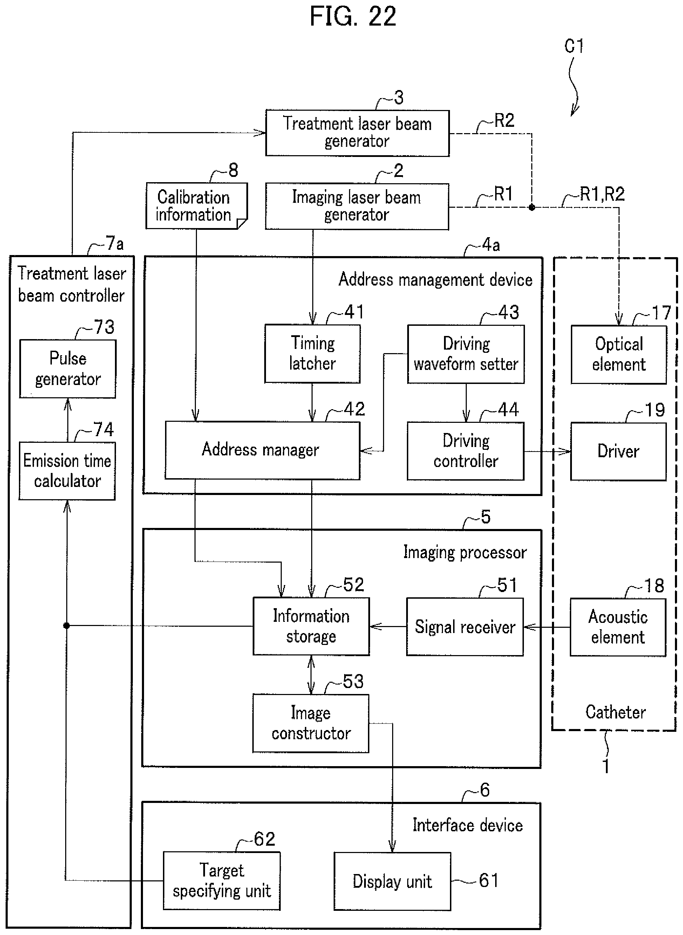

FIG. 22 is a functional block diagram of a photoacoustic catheter system C1 according to a second embodiment. Note that in FIG. 22, the same components as those in FIG. 3 are denoted by the same reference numerals, and descriptions thereof are omitted. The photoacoustic catheter system C1 in FIG. 22 differs from the photoacoustic catheter system C in FIG. 3 on the following three points:

(1) In the address management device 4a, the corrector 45 is omitted.
(2) In the address management device 4a, the calibration information 8 is inputted to the address manager 42. That is, the address manager 42 executes processing by the corrector 45 of the first embodiment. Note that the address management device 4a may include the corrector 45.
(3) In a treatment laser beam controller 7a, the address converter 71 and the comparator 72 are omitted. The treatment laser beam controller 7a has an emission time calculator 74. The emission time calculator 74 will be described later.

The photoacoustic catheter system C of the first embodiment emits the treatment laser beam R2 on the condition that an address (current address), at which imaging is currently in execution (the imaging laser beam R1 is emitted), matches an address (target address) specified as a target. In contrast, the photoacoustic catheter system C1 of the second embodiment emits the treatment laser beam R2 based on time management instead of address comparison.
More specifically, in the processing corresponding to step S1401 in FIG. 18, the emission time calculator 74 calculates an emission time of the treatment laser beam R2, based on the catheter-captured image, the address, the information about the area specified by the target specifying unit 62, and the like stored in the information storage 52.

That is, the photoacoustic catheter system C1 emits the treatment laser beam R2, based on the time calculated with the information about the area specified by the target specifying unit 62. In the processing corresponding to step S1402 in FIG. 18, the pulse generator 73 determines whether or not an emission time of the treatment laser beam has come. If an emission time of the treatment laser beam has come, the pulse generator 73 generates a pulse signal (processing corresponding to step S403 in FIG. 18). Then, the treatment laser beam generator 3 generates the treatment laser beam R2 (high power pulse laser beam) according to the pulse signal (processing corresponding to step S1404 in FIG. 18).

According to the second embodiment, components of the address management device 4a and the treatment laser beam controller 7a can be reduced to achieve cost reduction.

**Third Embodiment**

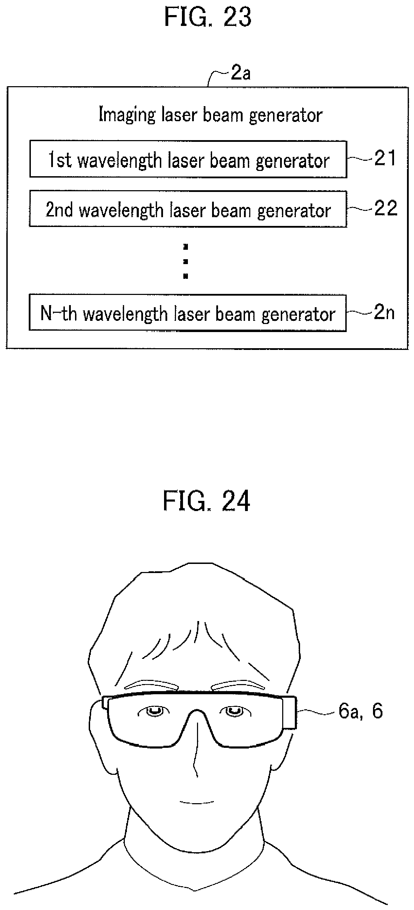

FIG. 23 is a diagram of an imaging laser beam generator 2a used in a third embodiment. As shown in FIG. 23, the imaging laser beam generator 2a includes a first wavelength laser beam generator 21, a second wavelength laser beam generator 22, - - -, an n-th wavelength laser beam generator 2n. The first wavelength laser beam generator 21, the second wavelength laser beam generator 22, - - -, the n-th wavelength laser beam generator 2n generate laser beams having different wavelengths, respectively. Laser beams having these wavelengths are mixed in the optical fiber 13 to travel therethrough. That is, a multicolor laser beam is emitted from the front end of the optical fiber 13.

Living tissues have different light absorption rates depending on the kind thereof. Therefore, the kind of the living tissue can be identified by irradiating a living tissue with a multicolor laser beam as shown in FIG. 23, and then distributing the difference in light absorption rate. This allows for identifying the lesion, or the like. In particular, lipid and a calcification area can be identified. This also allows for displaying an image, which distinguishes a healthy area from an area requiring treatment, on the display unit 61 through which an area to be treated can be specified. That is, the image constructor 53 determines a healthy area and an area requiring treatment, based on the difference in the light absorption rate of the living tissue, and displays the determined image on the display unit 61. This allows the user (physician) to easily determine a healthy area and an area requiring treatment, to improve the efficiency of treatment. In addition, the treatment laser beam controller 7 can implement a function that determines whether or not the area specified as an area to be treated is a healthy area, based on the difference in the living tissue (the difference in the light absorption rate of the living tissue), and avoids (prohibits) irradiating a healthy area with the treatment laser beam R2. In this manner, it can prevent an area, which requires no treatment, from being treated by mistake.

**Fourth Embodiment**

FIG. 24 is a diagram illustrating an example of the interface device 6 used in the present embodiment. As shown in FIG. 24, the interface device 6 may be a glasses-type wearable terminal 6a or the like, for example. In a case where the interface device 6 is composed of a PC screen and a pointing device, a physician cannot directly touch these devices during surgery. Therefore, a nurse operates the pointing device according to the instruction from the physician to specify the target. In contrast, in a case where the physician wears the glasses-type wearable terminal 6a, the physician can specify the target by himself/herself. This allows for improving the accuracy of treatment using the catheter 1 and shortening surgical time. In the case where the interface device 6 is the glasses-type wearable terminal 6a, a lesion may be specified with a particular eye movement.

Alternatively, the interface device 6 may be a head mounted display. Then, the physician may proceed with the treatment while viewing the catheter-captured image displayed on the head mounted display.

**Modifications**

Note that in the above-described embodiments, the catheter 1 emits a laser beam forward, but the present invention is not limited thereto. For example, a mirror in a cone shape may be arranged in the direction toward which a laser beam is emitted from the front end of the optical fiber 13, to allow a catheter to laterally emit the laser beam.

In addition, in the above-described embodiments, the treatment laser beam R2 is emitted while imaging is in operation, but the present invention is not limited thereto. That is, imaging may be separated from treatment to avoid imaging at the stage when the treatment laser beam R2 is emitted.

Note that a power adjuster may be provided in the interface device 6 or the like for varying power of the treatment laser beam R2 depending on the lesion. For example, if the lesion cannot be easily removed, the operator may raise the power of the treatment laser beam R2.

The present invention is not limited to the above-described embodiments, and includes various modificatios. For example, the above-described embodiments have been described in detail for the purpose of illustrating the present invention, and are not necessarily limited to those having all the components as described above. Also, a part of the configuration of an embodiment can be replaced with a configuration of another embodiment, or the configuration of an embodiment can be added with the configuration of another embodiment. Additionally, a part of the configuration of each embodiment may be deleted, or added/replaced with other configuration.

In addition, some or all of the above-described configurations, functions, components 41 to 45, 51, 53, 71 to 73, the information storage 52 and the like may be designed on an integrated circuit, for example, to implement them by hardware. Alternatively, programs for implementing the above-described configurations, functions, and the like may be interpreted and executed by a processor such as a CPU, to implement them by software. Information such as programs for implementing respective functions, tables, and files can be stored in a recording device such as a memory device and an SSD (Solid State Drive), or a recording media such as an IC (Integrated Circuit) card, an SD (Secure Digital) card, and a DVD (Digital Versatile Disc), in addition to storing in an HD (Hard Disk). Further, in each embodiment, the control line and the information line indicate what is/are considered to be necessary for the purpose of illustration, but may not necessarily indicate all the control lines and information lines for the respective products. In fact, it is safe to assume that almost all components are connected with one another.

LEGEND FOR REFERENCE NUMERALS

| 1 | Catheter |
|---|---|
| 2 | Imaging laser beam generator |
| 3 | Treatment laser beam generator |
| 4 | Address management device (Controller) |
| 5 | Imaging processor |
| 6 | Interface device (Input/output device) |
| 6a | Wearable terminal |
| 7 | Treatment laser beam controller |
| 8 | Calibration information (distortion information) |
| 11 | Acoustic element (Acoustic detector) |
| 13 | Optical fiber |
| 14 | Driver |
| 15 | Cover |
| 16 | Lens |
| 17 | Optical element |
| 18 | Acoustic element (Acoustic detector) |
| 19 | Driver |
| 21 | First wavelength laser beam generator |
| 22 | Second wavelength laser beam generator |
| 2n | N-th wavelength laser beam generator |
| 41 | Timing latcher |
| 42 | Address manager |
| 43 | Driving waveform generator |
| 44 | Driving controller |
| 45 | Corrector |
| 51 | Signal receiver |
| 52 | Information storage |
| 53 | Image constructor |
| 61 | Display unit |
| 62 | Target specifying unit |
| 71 | Address converter |
| 72 | Comparator |
| 73 | Pulse generator |
| 74 | Emission time calculator |
| 101 | Trail |
| C, C1 | Photoacoustic catheter system (including liquid injector) |

**Claims**

**1.** A photoacoustic catheter system comprising:

an imaging laser beam generator that generates an imaging laser beam used for imaging;
a treatment laser beam generator that generates a treatment laser beam used for treatment;
an emitter that emits the imaging laser beam and the treatmen laser beam so as to be directionally aligned with each other;
a driver that drives the emitter so as to emit the imaging laser beam and the treatment laser beam toward a predetermined direction with respect to an advancing direction of a catheter;
an acoustic detector that receives an acoustic wave generated due to irradiation of the imaging laser beam; and
a controller that causes the emitter to synchronously emit the imaging laser beam and the treatment laser beam.

2. The photoacoustic catheter system according to claim 1, further comprising:

an input/output device that displays an image captured with the imaging laser beam so that an area to be treated can be specified therethrough,

wherein the controller causes the treatment laser beam generator to emit the treatment laser beam so that an area specified as the area to be treated is irradiated with the treatment laser beam.

3. The photoacoustic catheter system according to claim 2,
wherein the input/output device is a wearable terminal.

4. The photoacoustic catheter system according to claim 1, further comprising:

an input/output device that displays an image captured with the imaging laser beam so that an area to be treated can be specified therethrough,

wherein the controller causes the treatment laser beam generator to emit the treatment laser beam, based on a time calculated with information about the area specified via the input/output device.

5. The photoacoustic catheter system according to claim 1,
wherein the imaging laser beam and the treatment laser beam travel through a single optical fiber as the emitter, the acoustic detector is arranged in a ring shape at an end of the optical fiber from which the imaging laser beam and the treatment laser beam are emitted, and
a front end of the optical fiber as the emitter is caused by the driver to emit the imaging laser beam and the treatment laser beam through a hollow in the acoustic detector arranged in a ring shape, so as to draw a trail in a spriral shape.

6. The photoacoustic catheter system according to claim 5,
wherein the controller has distortion information about distortion of an emission position of the imaging laser beam, and calibrates an image captured with the imaging laser beam according to the distortion information.

7. The photoacoustic catheter system according to claim 1,
wherein the imaging laser beam is composed of laser beams having a plurality of frequencies.

8. The photoacoustic catheter system according to claim 1,
wherein a core for introducing the treatment laser beam into an optical fiber as the emitter is separately arranged from a core for introducing the imaging laser beam.

9. The photoacoustic catheter system according to claim 1,
wherein the treatment laser beam is the same laser beam as the imaging laser beam, and power is changed to switch the imaging laser beam and the treatment laser beam.

10. The photoacoustic catheter system according to claim 1,
wherein at least one of intensity of the treatment laser beam and duration of treatment can be set based on information entered via an input/output device.

11. The photoacoustic catheter system according to claim 2,
wherein the imaging laser beam is composed of laser beams having a plurality of frequencies, and
an image, which distinguishes a healthy area from an area requiring treatment, is displayed on the input/output device through which an area to be treated can be specified.

**12.** The photoacoustic catheter system according to claim 2,
wherein the imaging laser beam is composed of laser beams having a plurality of frequencies, and
if an area specified as the area to be treated is found to be a healthy area, said area is not irradiated with the
treatment laser beam.

**13.** The photoacoustic catheter system according to claim 1,
wherein the acoustic detector is of an array type having a plurality of elements.

**14.** The photoacoustic catheter system according to claim 1, further comprising:

a liquid injector that is used to inject a transparent liquid for provisionally removing blood into an optical fiber as
the emitter.

**15.** A control method of a photoacoustic catheter system, for use in a photoacoustic catheter system including: an
imaging laser beam generator that generates an imaging laser beam used for imaging; a treatment laser beam
generator that generates a treatment laser beam used for treatment; an emitter that emits the imaging laser beam
and the treatmen laser beam so as to be directionally aligned with each other; a driver that drives the emitter so as
to emit the imaging laser beam and the treatment laser beam toward a predetermined direction with respect to an
advancing direction of a catheter; and an acoustic detector that receives an acoustic wave generated due to irradiation
of the imaging laser beam,
the method comprising:

emitting the imaging laser beam;
receiveing an acoustic wave generated due to irradiation of the imaging laser beam;
displaying a catheter-captured image on a display unit;
identifying a lesion; and
irradiate the lesion with the treatment laser beam,

wherein the imaging laser beam and the treatment laser beam are synchronously emitted from the emitter.

# FIG. 1

# FIG. 2

# FIG. 3

C

```
                        ┌─────────────────────┐
                        │  Treatment laser beam│ ～3      ～R2
                        │     generator        │
                        └─────────────────────┘
                                                            ～R1,R2
        ～8              ～2
┌──────────────┐  ┌─────────────────────┐  ～R1
│ Calibration  │  │ Imaging laser beam   │
│ information   │  │     generator        │
└──────────────┘  └─────────────────────┘
```

| ～7 Treatment laser beam controller | ～4 Address management device | Catheter |
|---|---|---|

```
Treatment laser          Address management device               ～17
beam controller                                              ┌──────────┐
    ～73                ～41            ～43                   │ Optical  │
┌──────────┐        ┌─────────┐  ┌──────────────┐           │ element  │
│  Pulse   │        │ Timing  │  │   Driving    │           └──────────┘
│generator │        │ latcher │  │waveform setter│
└──────────┘        └─────────┘  └──────────────┘               ～19
    ～72      ～45        ～42          ～44               ┌──────────┐
┌──────────┐ ┌────────┐ ┌─────────┐ ┌──────────┐         │  Driver  │
│Comparator│◄│Corrector│ │ Address │ │ Driving  │         └──────────┘
└──────────┘ └────────┘ │ manager │◄│controller│
    ～71                 └─────────┘ └──────────┘
┌──────────┐
│ Address  │              ～5
│converter │        Imaging processor
└──────────┘
                    ～52          ～51                          ～18
             ┌─────────────┐ ┌──────────────┐           ┌──────────┐
             │ Information  │◄│Signal receiver│◄          │ Acoustic │
             │  storage     │ └──────────────┘           │ element  │
             └─────────────┘                             └──────────┘
                    ～53
             ┌─────────────┐
             │    Image     │                             Catheter
             │ constructor  │                                 1
             └─────────────┘
```

```
                        ～6
                   Interface device
   ～62
┌──────────────┐        ┌──────────────┐
│    Target     │        │ Display unit │～61
│specifying unit│        └──────────────┘
└──────────────┘
```

# FIG. 4

```
        ( START )
            │
            ▼
    ┌─────────────────┐
    │    Activate     │ ── S1
    └─────────────────┘
            │
            ▼
    ┌─────────────────┐
    │ Imaging laser beam │ ── S2
    │ emission processing │
    └─────────────────┘
            │
            ▼
    ┌ ─ ─ ─ ─ ─ ─ ─ ─ ┐
      Acoustic wave is generated  ── S3
    └ ─ ─ ─ ─ ─ ─ ─ ─ ┘
            │
            ▼
    ┌─────────────────┐
    │ Detect acoustic wave │ ── S4
    └─────────────────┘
            │
            ▼
    ┌─────────────────┐
    │ Address correction │ ── S5
    │    processing    │
    └─────────────────┘
            │
            ▼
    ┌─────────────────┐
    │ Image processing │ ── S6
    └─────────────────┘
            │
            ▼
    ┌─────────────────┐
    │    Display      │ ── S7
    │ catheter-captured image │
    └─────────────────┘
            │
            ▼       S11
       ╱ Has lesion ╲   No
      ╱  been identified? ╲────────┐
       ╲               ╱           │
        ╲    Yes     ╱             ▼        S12
            │              ╱ Will lesion ╲    No
            │             ╱  be identified? ╲─────→
            │              ╲              ╱
            │                  ╲  Yes   ╱
            │                      │         S13
            │              ┌─────────────────┐
            │              │ Lesion identification processing │
            │              └─────────────────┘
            │                      │
            ▼◄─────────────────────┘
    ┌─────────────────┐
    │ Treatment laser beam │ ── S14
    │ emission processing │
    └─────────────────┘
```

# FIG. 5

```
        ( START )
            │
            ▼
┌──────────────────────┐
│   Set driving voltage │ ─ S201
│       waveform        │
└──────────────────────┘
            │
            ▼
┌──────────────────────┐
│      Generate         │ ─ S202
│   driving voltage     │
└──────────────────────┘
            │
            ▼
┌──────────────────────┐
│       Apply           │ ─ S203
│   driving voltage     │
└──────────────────────┘
            │
            ▼
┌──────────────────────┐
│ Record emission timing│ ─ S204
└──────────────────────┘
            │
            ▼
┌──────────────────────┐
│     Store address     │ ─ S205
└──────────────────────┘
            │
            ▼
        ( RETURN )
```

## FIG. 6

EP 3 427 641 A1

FIG. 7

14,19

D3, D

D4, D

D2, D

13

D1, D

# FIG. 8

## FIG. 9

## FIG. 10

# FIG. 11

```
        ( START )
            │
            ▼
┌─────────────────────────────┐
│ Obtain calibration information │──S501
└─────────────────────────────┘
            │
            ▼
┌─────────────────────────────┐
│  Calculate corrected address  │──S502
└─────────────────────────────┘
            │
            ▼
       ( RETURN )
```

# FIG. 12

# FIG. 13

## FIG. 14

```
              START

   Convert voltage signal into image signal    S601

      Calculate distance and direction         S602

      Generate catheter-captured image         S603

              RETURN
```

# FIG. 15

# FIG. 16

# FIG. 17

```
        ( START )
            │
            ▼
┌───────────────────────────────┐
│ Display catheter-captured image │──S1301
└───────────────────────────────┘
            │
            ▼
┌───────────────────────────────┐
│       Target is specified       │──S1302
└───────────────────────────────┘
            │
            ▼
        ( RETURN )
```

# FIG. 18

```
        ( START )
            │
            ▼
┌───────────────────────────────┐
│     Calculate target address    │──S1401
└───────────────────────────────┘
            │
            ▼       ┌─S1402
         ╱───────────────────────╲
        ╱     Does                 ╲    No
       ⟨ target address match current ⟩────┐
        ╲        address ?          ╱      │
         ╲───────────────────────╱         │
            │ Yes                          │
            ▼                              │
┌───────────────────────────────┐         │
│       Generate pulse signal     │──S1403 │
└───────────────────────────────┘         │
            │                              │
            ▼                              │
┌───────────────────────────────┐         │
│    Emit treatment laser beam    │──S1404 │
└───────────────────────────────┘         │
            │
            ▼
        ( RETURN )
```

FIG. 19

EP 3 427 641 A1

FIG. 20

## FIG. 21

401

# FIG. 22

C1

Treatment laser beam generator ~3

~R2

Calibration information ~8

Imaging laser beam generator ~2

~R1

~R1,R2

Treatment laser beam controller ~7a

Pulse generator ~73

Emission time calculator ~74

Address management device ~4a

Timing latcher ~41

Driving waveform setter ~43

Optical element ~17

Address manager ~42

Driving controller ~44

Driver ~19

Imaging processor ~5

Information storage ~52

Signal receiver ~51

Acoustic element ~18

Image constructor ~53

Catheter

1

Interface device ~6

Target specifying unit ~62

Display unit ~61

## FIG. 23

~2a

| Imaging laser beam generator |
| --- |
| 1st wavelength laser beam generator  ~21 |
| 2nd wavelength laser beam generator  ~22 |
| ⋮ |
| N-th wavelength laser beam generator  ~2n |

## FIG. 24

6a, 6

Europäisches
Patentamt

European
Patent Office

Office européen
des brevets

# EUROPEAN SEARCH REPORT

Application Number

EP 18 16 2336

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y<br>A | DE 199 16 653 A1 (LUBATSCHOWSKI HOLGER [DE]) 19 October 2000 (2000-10-19)<br>* column 2, line 54 - column 4, line 29 *<br>* figures *<br>* claim 6 * | 1-5, 7-12,15<br>6<br>13,14 | INV.<br>A61B5/00 |
| X | JP 2017 080440 A (FUJIFILM CORP) 18 May 2017 (2017-05-18)<br>* paragraphs [0135] - [0155] * | 1-5, 7-12,15 | |
| X | US 2017/112384 A1 (MASWADI SAHER [US] ET AL) 27 April 2017 (2017-04-27)<br>* paragraphs [0006] - [0011], [0025] - [0035] * | 1-5, 7-12,15 | |
| X | US 2010/280504 A1 (MANZKE ROBERT [US] ET AL) 4 November 2010 (2010-11-04)<br>* paragraphs [0011] - [0023] * | 1-5, 7-13,15 | |
| X | US 2012/271170 A1 (EMELIANOV STANISLAV [US] ET AL) 25 October 2012 (2012-10-25)<br>* paragraphs [0051] - [0058] *<br>* claims * | 1-5, 7-13,15 | TECHNICAL FIELDS SEARCHED (IPC)<br><br>A61B |
| X | US 2015/038824 A1 (LUPOTTI FERMIN A [US]) 5 February 2015 (2015-02-05)<br>* paragraphs [0009] - [0013], [0029] - [0039], [0056] - [0061] * | 1-5,7-15 | |
| Y | WO 2013/188707 A1 (SENO MEDICAL INSTR INC [US]) 19 December 2013 (2013-12-19)<br>* paragraphs [0131] - [0154] * | 6 | |

The present search report has been drawn up for all claims

| Place of search | Date of completion of the search | Examiner |
|---|---|---|
| Munich | 18 September 2018 | Lohmann, Stefan |

EPO FORM 1503 03.82 (P04C01)

## ANNEX TO THE EUROPEAN SEARCH REPORT
## ON EUROPEAN PATENT APPLICATION NO.

EP 18 16 2336

This annex lists the patent family members relating to the patent documents cited in the above-mentioned European search report.
The members are as contained in the European Patent Office EDP file on
The European Patent Office is in no way liable for these particulars which are merely given for the purpose of information.

18-09-2018

| Patent document cited in search report | | Publication date | Patent family member(s) | | Publication date |
|---|---|---|---|---|---|
| DE 19916653 | A1 | 19-10-2000 | NONE | | |
| JP 2017080440 | A | 18-05-2017 | JP 6066232 B2 | | 25-01-2017 |
| | | | JP 6066233 B2 | | 25-01-2017 |
| | | | JP 6291019 B2 | | 14-03-2018 |
| | | | JP 2015231582 A | | 24-12-2015 |
| | | | JP 2015231583 A | | 24-12-2015 |
| | | | JP 2017080440 A | | 18-05-2017 |
| | | | JP 2018089406 A | | 14-06-2018 |
| US 2017112384 | A1 | 27-04-2017 | NONE | | |
| US 2010280504 | A1 | 04-11-2010 | AT 511787 T | | 15-06-2011 |
| | | | CN 101909516 A | | 08-12-2010 |
| | | | EP 2229096 A1 | | 22-09-2010 |
| | | | JP 5698538 B2 | | 08-04-2015 |
| | | | JP 2011507651 A | | 10-03-2011 |
| | | | RU 2010131476 A | | 10-02-2012 |
| | | | US 2010280504 A1 | | 04-11-2010 |
| | | | WO 2009083859 A1 | | 09-07-2009 |
| US 2012271170 | A1 | 25-10-2012 | US 2012271170 A1 | | 25-10-2012 |
| | | | WO 2011053931 A2 | | 05-05-2011 |
| US 2015038824 | A1 | 05-02-2015 | CN 104114098 A | | 22-10-2014 |
| | | | EP 2814397 A1 | | 24-12-2014 |
| | | | JP 6038957 B2 | | 07-12-2016 |
| | | | JP 2015513350 A | | 11-05-2015 |
| | | | US 2015038824 A1 | | 05-02-2015 |
| | | | WO 2013123014 A1 | | 22-08-2013 |
| WO 2013188707 | A1 | 19-12-2013 | AU 2013274136 A1 | | 11-12-2014 |
| | | | AU 2018201462 A1 | | 22-03-2018 |
| | | | CA 2874874 A1 | | 19-12-2013 |
| | | | EP 2861152 A1 | | 22-04-2015 |
| | | | JP 6239604 B2 | | 29-11-2017 |
| | | | JP 2015519183 A | | 09-07-2015 |
| | | | KR 20150023241 A | | 05-03-2015 |
| | | | SG 11201407748S A | | 29-01-2015 |
| | | | WO 2013188707 A1 | | 19-12-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016063406 A